# EUROPEAN PATENT APPLICATION

(11) **EP 4 488 391 A1**
(43) Date of publication of application: **08.01.2025**
(21) Application number: 23183416.9
(22) Date of filing: 04.07.2023
(51) Int. Cl.: C12Q 1/689

(54) **METHODS AND MEANS FOR DETECTION OF LEGIONELLA**

(71) Applicant: Nederlandse Organisatie voor toegepast-natuurwetenschappelijk Onderzoek TNO, 2595 DA 's-Gravenhage (NL)
(72) Inventor: VAN DER WURFF, Michelle, 2595 DA 's-Gravenhage (NL); KEIJSER, Bart Jan Frederik, 2595 DA 's-Gravenhage (NL); KIEBOOM, Jasper, 2595 DA 's-Gravenhage (NL); DEKKER, Floris Anthonie Jan, 2595 DA 's-Gravenhage (NL); VERMEULEN, Kim, 2595 DA 's-Gravenhage (NL)
(74) Representative: V.O.

(57) **Abstract**

The invention relates to methods for determining whether a sample comprises *Legionella pneumophila,* the method comprising performing an isothermal nucleic acid amplification (iNAAT) reaction, preferably loop-mediated isothermal amplification (LAMP), with said sample with a primer set specific for the *Legionella pneumophila* DotB or MIP gene and determining whether the sample comprises an amplification product of the amplification reaction. The invention further relates to primer sets specific for the *Legionella pneumophila* DotB or MIP gene that are useful in such method, and kits of part comprising such primer set.

## Description

### Field of the invention

The invention relates to methods, primers and kits for the detection of *Legionella* spp, in particular *Legionella pneumophila,* in particular for isothermal nucleic acid amplification (iNAAT) based detection thereof.

### Background of the invention

*Legionella* bacteria are rod-shaped non-spore forming bacteria that are ubiquitous in water and soil. The genus holds 60 known species, and although several *Legionella* species have been associated with human infection, *Legionella pneumophila* is the prime cause of Legionnaire's disease and the most common cause of Pontiac fever. The bacteria can grow in warm, stagnant water systems such as those in cooling towers, water distribution systems within public buildings, households and industrial facilities and hydric pipelines in hospitals. The main route for transmission is through inhalation of small droplets (aerosols), posing a health risk for the population. The risk associated with *Legionella pneumophila* is exemplified by regular deadly outbreaks.

Legislation for proper control of legionella is in place to safeguard risks in many countries. This typically involves technical measures for prevention and biannual surveillances for risk environments (e.g. hotels, hospitals, camp sites and swimming pools) by legionella testing. In clinical practice, *Legionella pneumophila* testing is relevant for the correct diagnosis of disease.

*Legionella* testing is performed both on clinical samples as well as on environmental (in particular water) samples. Diagnostic practices include bacterial culture, serological and antibody-based assays, nucleic acid detection systems (e.g., polymerase chain reaction) and urine antigen tests.

Current testing methods rely both on cultivation as well as on PCR-based molecular detection. In the clinic, also antigen and antibody based testing is performed, reflecting the immune response of the patient to a previous *Legionella* infection. Cultivation-dependent analysis takes between 10-14 days, molecular testing requires 24-72 hours. While PCR is relatively rapid, providing results in approximately 1-3 hours, shipment to a molecular microbiology laboratory and sample handling delays provision of results.

The current gold standard in molecular diagnosis of Legionella involves the detection of the MIP gene, which is specific to Legionella pneumophila, and the 16S rRNA gene, which is used for identifying the Legionella genus.

Loop mediated isothermal amplification (LAMP), which allows on-site analysis, has been deployed for the detection of *Legionella* spp and *Legionella pneumophila.* EP3187595 describes a method for LAMP designed to amplify specifically sequences of Legionella 's 16S ribosomal RNA (rRNA) gene using a set of specially designed LAMP primers. CN106755302A describes a LAMP primer set for the DotA gene of *Legionella pneumophila.* CN105483211A describes a nested LAMP primer set for *Legionella pneumophila* detection primers specific for the MIP gene.

There is a need in the art to provide further *Legionella* detection methods which further enhance the speed, ease, sensitivity and/or broad taxonomic coverage of *Legionella* detection.

### Summary of the invention

It is an object of the present invention to provide reliable, sensitive and fast *Legionella* detection.

The invention therefore provides a method for determining whether a sample comprises *Legionella pneumophila*, the method comprising performing an isothermal nucleic acid amplification (iNAAT) reaction with said sample with a primer set specific for the *Legionella pneumophila* DotB gene and determining whether the sample comprises an amplification product of the amplification reaction.

In a further aspect, the invention provides a method of detecting an amplification product in a sample, the method comprising performing an isothermal nucleic acid amplification (iNAAT) reaction with said sample with a primer set specific for the *Legionella pneumophila* DotB gene and determining whether the sample comprises an amplification product of the amplification reaction.

In a further aspect, the invention provides a method for determining whether a sample comprises *Legionella pneumophila*, the method comprising performing an isothermal nucleic acid amplification (iNAAT) reaction with said sample with a primer set specific for the *Legionella pneumophila* MIP gene, wherein said primer set comprises a forward outer primer (F3), a backward outer primer (B3), a forward inner primer (FIP) and a backward inner primer (BIP), and optionally further comprising a loop forward primer (LoopF) and/or loop backward primer (LoopB), preferably at least one Loop primer, preferably a LoopB primer, and determining whether the sample comprises an amplification product of the amplification reaction, wherein:
- primer F3 comprises a consecutive stretch of at least 15 nucleotides of the sequence CAAAGTAATCAATTCTGGAAATGG;
- primer B3 comprises a consecutive stretch of at least 15 nucleotides of the sequence ACATAAATTTCCCAAGTTGATCC;
- primer FIP comprises a consecutive stretch of at least 30 nucleotides or two consecutive stretches of at least 30 nucleotides in total of the sequence GTACCATCAATCAGACGACCAGGTTAAACCCGGAAAATCGGA;
- primer BIP comprises a consecutive stretch of at least 30 nucleotides or two consecutive stretches of at least 30 nucleotides in total of the sequence CCGAAAAAACTGGTAAGCCAGCTGGCATCAATTGCAAAGC, and
   optionally
- primer LoopB comprises a consecutive stretch of at least 15 nucleotides of the sequence CAAGTTATCCCTGGATGGACAGAA.

In a further aspect, the invention provides a method of detecting an amplification product in a sample, the method comprising performing an isothermal nucleic acid amplification (iNAAT) reaction with said sample with a primer set specific for the *Legionella pneumophila* MIP gene, wherein said primer set comprises a forward outer primer (F3), a backward outer primer (B3), a forward inner primer (FIP) and a backward inner primer (BIP), and optionally further comprising a loop forward primer (LoopF) and/or loop backward primer (LoopB), preferably at least one Loop primer, preferably a LoopB primer, and determining whether the sample comprises an amplification product of the amplification reaction, wherein
- primer F3 comprises a consecutive stretch of at least 15 nucleotides of the sequence CAAAGTAATCAATTCTGGAAATGG;
- primer B3 comprises a consecutive stretch of at least 15 nucleotides of the sequence ACATAAATTTCCCAAGTTGATCC;
- primer FIP comprises a consecutive stretch of at least 30 nucleotides or two consecutive stretches of at least 30 nucleotides in total of the sequence GTACCATCAATCAGACGACCAGGTTAAACCCGGAAAATCGGA;
- primer BIP comprises a consecutive stretch of at least 30 nucleotides or two consecutive stretches of at least 30 nucleotides in total of the sequence CCGAAAAAACTGGTAAGCCAGCTGGCATCAATTGCAAAGC, and
   optionally
- primer LoopB comprises a consecutive stretch of at least 15 nucleotides of the sequence CAAGTTATCCCTGGATGGACAGAA.

In preferred embodiments, a method of the invention comprises performing an isothermal nucleic acid amplification (iNAAT) reaction with said sample with a primer set specific for the *Legionella pneumophila* MIP gene according to the invention and a primers set specific for the a *Legionella pneumophila* DotB gene according to the invention.

In preferred embodiments, a MIP primer set according to the invention and a DotB primer set according to the invention are combined. Preferably, both a MIP primer set according to the invention and a DotB primer set according to the invention are included in a kit of the invention or used in a method of the invention.

In a further aspect, the invention provides a loop-mediated isothermal amplification (LAMP) primer set specific for *Legionella pneumophila* DotB gene, the primer set comprising a forward outer primer (F3), a backward outer primer (B3), a forward inner primer (FIP) and a backward inner primer (BIP), and optionally a loop forward primer (LoopF) and/or loop backward primer (LoopB), preferably at least one Loop primer, preferably a LoopF primer.

In a further aspect, the invention provides a loop-mediated isothermal amplification (LAMP) primer set specific for *Legionella pneumophila* MIP gene, the primer set comprising a forward outer primer (F3), a backward outer primer (B3), a forward inner primer (FIP) and a backward inner primer (BIP), and optionally a loop forward primer (LoopF) and/or loop backward primer (LoopB), wherein:
- primer F3 comprises a consecutive stretch of at least 15 nucleotides of the sequence CAAAGTAATCAATTCTGGAAATGG;
- primer B3 comprises a consecutive stretch of at least 15 nucleotides of the sequence ACATAAATTTCCCAAGTTGATCC;
- primer FIP comprises a consecutive stretch of at least 30 nucleotides or two consecutive stretches of at least 30 nucleotides in total of the sequence GTACCATCAATCAGACGACCAGGTTAAACCCGGAAAATCGGA;
- primer BIP comprises a consecutive stretch of at least 30 nucleotides or two consecutive stretches of at least 30 nucleotides in total of the sequence CCGAAAAAACTGGTAAGCCAGCTGGCATCAATTGCAAAGC, and
   optionally
- primer LoopB comprises a consecutive stretch of at least 15 nucleotides of the sequence CAAGTTATCCCTGGATGGACAGAA.

In a further aspect, the invention provides a kit of parts comprising a LAMP primer set according to the invention.

In preferred embodiments, the kit of parts comprises a primer set specific for the *Legionella pneumophila* MIP gene according to the invention and a primers set specific for the a *Legionella pneumophila* DotB gene according to the invention.

In a further aspect, the invention provides a use of a primer set or kit of parts according to the invention for determining whether a sample comprises *Legionella pneumophila*, in particular using a method according to the invention.

In a further aspect, the invention provides a use of a primer set or kit of parts according to the invention for detecting *Legionella pneumophila* in a sample.

In a further aspect, the invention provides a use of a primer set or kit of parts according to the invention for detecting an amplification product of *Legionella pneumophila* nucleic acid in a sample.

### Detailed description

As used herein, "to comprise" and its conjugations is used in its non-limiting sense to mean that items following the word are included, but items not specifically mentioned are not excluded. In addition the verb "to consist" may be replaced by "to consist essentially of" meaning that a compound or adjunct compound as defined herein may comprise additional component(s) than the ones specifically identified, said additional component(s) not altering the unique characteristic of the invention.

The articles "a" and "an" are used herein to refer to one or to more than one (i.e., to at least one) of the grammatical object of the article. By way of example, "an element" means one element or more than one element.

The word "approximately" or "about" when used in association with a numerical value (approximately 10, about 10) preferably means that the value may be the given value of 10 more or less 1% of the value.

The use of the alternative (e.g., "or") should be understood to mean either one, both, or any combination thereof of the alternatives.

The present inventors have established novel LAMP based detection methods that enable rapid and sensitive detection of *Legionella pneumophila.* The method is fast, robust and sensitive in detecting *Legionella pneumophila.* An advantage of this method is that it abolishes the need for a sophisticated PCR machine. As the reaction takes place at a constant temperature, laboratory infrastructure can be made more simple and cheap. As the LAMP assay has been proven to be robust, the assay can even be translated to a set-up for analysis on site. A further advantage is the speed of the assay, that is completed within 15 minutes.

In a first aspect, the invention therefore provides a method for determining whether a sample comprises *Legionella pneumophila*, the method comprising performing an isothermal nucleic acid amplification (iNAAT) reaction with said sample with a primer set specific for the *Legionella pneumophila* DotB gene and determining whether the sample comprises an amplification product of the amplification reaction.

Also provided is a method of detecting an amplification product in a sample, in particular an amplification product of *Legionella pneumophila* nucleic acid, the method comprising performing an isothermal nucleic acid amplification (iNAAT) reaction with said sample with a primer set specific for the *Legionella pneumophila* DotB gene and determining whether the sample comprises an amplification product of the amplification reaction.

In a further aspect, the invention provides method for determining whether a sample comprises *Legionella pneumophila*, the method comprising performing an isothermal nucleic acid amplification (iNAAT) reaction with said sample with a primer set specific for the *Legionella pneumophila* MIP gene, wherein said primer set comprises a forward outer primer (F3), a backward outer primer (B3), a forward inner primer (FIP) and a backward inner primer (BIP), and optionally further comprising a loop forward primer (LoopF) and/or loop backward primer (LoopB), preferably at least one Loop primer, preferably a LoopB primer, and determining whether the sample comprises an amplification product of the amplification reaction, wherein:
- primer F3 comprises a consecutive stretch of at least 15 nucleotides of the sequence CAAAGTAATCAATTCTGGAAATGG;
- primer B3 comprises a consecutive stretch of at least 15 nucleotides of the sequence ACATAAATTTCCCAAGTTGATCC;
- primer FIP comprises a consecutive stretch of at least 30 nucleotides or two consecutive stretches of at least 30 nucleotides in total of the sequence GTACCATCAATCAGACGACCAGGTTAAACCCGGAAAATCGGA;
- primer BIP comprises a consecutive stretch of at least 30 nucleotides or two consecutive stretches of at least 30 nucleotides in total of the sequence CCGAAAAAACTGGTAAGCCAGCTGGCATCAATTGCAAAGC, and
   optionally
- primer LoopB comprises a consecutive stretch of at least 15 nucleotides of the sequence CAAGTTATCCCTGGATGGACAGAA.

Also provided is a method of detecting an amplification product in a sample, in particular an amplification product of *Legionella pneumophila* nucleic acid, the method comprising performing an isothermal nucleic acid amplification (iNAAT) reaction with said sample with a primer set specific for the *Legionella pneumophila* MIP gene, wherein said primer set comprises a forward outer primer (F3), a backward outer primer (B3), a forward inner primer (FIP) and a backward inner primer (BIP), and optionally further comprising a loop forward primer (LoopF) and/or loop backward primer (LoopB), preferably at least one Loop primer, preferably a LoopB primer, and determining whether the sample comprises an amplification product of the amplification reaction, wherein
- primer F3 comprises a consecutive stretch of at least 15 nucleotides of the sequence CAAAGTAATCAATTCTGGAAATGG;
- primer B3 comprises a consecutive stretch of at least 15 nucleotides of the sequence ACATAAATTTCCCAAGTTGATCC;
- primer FIP comprises a consecutive stretch of at least 30 nucleotides or two consecutive stretches of at least 30 nucleotides in total of the sequence GTACCATCAATCAGACGACCAGGTTAAACCCGGAAAATCGGA;
- primer BIP comprises a consecutive stretch of at least 30 nucleotides or two consecutive stretches of at least 30 nucleotides in total of the sequence CCGAAAAAACTGGTAAGCCAGCTGGCATCAATTGCAAAGC, and
   optionally
- primer LoopB comprises a consecutive stretch of at least 15 nucleotides of the sequence CAAGTTATCCCTGGATGGACAGAA.

A method of the invention comprises performing an isothermal nucleic acid amplification (iNAAT) reaction with a sample. "iNAAT" refer to Isothermal Nucleic Acid Amplification Technology. "LAMP" is one example of an iNAAT reaction and refers to Loop-mediated Isothermal Amplification. Other examples of iNAAT include strand-displacement amplification (SDA), helicase-dependent amplification (HDA) and recombinase polymerase amplification (RPA). iNAAT and LAMP reactions proceed at a constant temperature using a strand displacement reaction, as opposed to e.g. PCR amplification method wherein an amplification reaction comprises multiple cycles, each cycle consisting of multiple stages performed at different temperatures.

In preferred embodiments, the isothermal nucleic acid amplification reaction performed in a method of the invention is a LAMP reaction.

LAMP uses 4-6 primers recognizing 6-8 distinct regions of the target sequence. A strand-displacing DNA polymerase initiates synthesis and 2 of the primers form loop structures for subsequent rounds of amplification. The 4 required primers are a forward outer primer (F3), a backward outer primer (B3), a forward inner primer (FIP) and a backward inner primer (BIP). Optional primers are a loop forward primer (LoopF) and a loop backward primer (LoopB). The F3 and B3 primers are (complementary to) a 3' and 5' region in the target sequence. The FIP and BIP are primers that comprise two stretches of nucleotides that are complementary to two different regions of the target sequence, which regions are not located directly adjacent to each other in the target sequence. The FIP and BIP form the loop structures for subsequent rounds of amplification. The LoopF and LoopB primers can be used to further accelerate the reaction.

The skilled person will know how to conduct an amplification method and which reaction conditions to use. For example, a suitable way of conducting the LAMP method is described in EP1020534A1 and EP1327679A1. Further, the Examples herein provide an exemplary LAMP method.

As used herein, the term "sample" as used in its broadest sense to refer to any biological sample from any human or veterinary subject or any environmental sample that may be tested for the presence or absence of *Legionella pneumophila* nucleic acid. "Biological sample" as used herein refers to a sample obtained from an organism, including tissue, cells, stool or fluid. Biological samples may include, without limitation, any fluid including blood, plasma, serum, urine, lymphatic fluid, synovial fluid, cerebrospinal fluid, amniotic fluid, amniotic cord blood, tears, saliva, and nasopharyngeal washes, or tissues obtained from any organ. The biological sample can also be a stool sample or a cell or cell culture sample. "Environmental sample" as used herein a sample of any material collected from environmental sources such as air, water or soil or any other natural sample. Preferred examples of environmental samples include water samples, e.g. obtained from an industrial, health-care or residential facility or setting, from facilities such as hotels, hospitals, camp sites and swimming pools, or from a natural setting. The sample is preferably a biological sample of an individual or an environmental sample. Preferably a biological sample is a body fluid sample such as whole blood, serum, plasma, urine, or sputum sample, more preferably whole blood, serum or plasma. Preferably an environmental sample is an environmental water sample.

As used herein, *"Legionella pneumophila* nucleic acid" refers to DNA or RNA. In preferred embodiments, the nucleic acid comprises or is DNA. DNA can be directly amplified with an iNAAT, preferably LAMP, reaction in a method of the invention, optionally after extraction, isolation and/or purification thereof. Alternatively, the nucleic acid comprises or is RNA. Detection of Legionella pneumophila (nucleic acid) is then based on a DotB transcript or MIP transcript, depending on the primer set used in the method of the invention. In this case reverse transcription is preferably performed. Reverse transcription is for instance performed in a reverse transcription loop-mediated isothermal amplification (RT-LAMP). RT-LAMP is a one-step nucleic acid amplification reaction that combines reverse transcription to synthesize cDNA from RNA with LAMP DNA amplification. Hence, in some embodiments, a method of the invention comprises a reverse transcription reaction, wherein *Legionella pneumophila* RNA is transcribed into cDNA. The reverse transcription reaction is performed using a reverse transcriptase, which enzymes are well known in the art and readily identifiable by a person skilled in the art. Alternatively, reverse transcription is performed in a separate reaction, preceding a method of the invention wherein an iNAAT reaction, preferably LAMP reaction, is performed.

For use in a method of the invention, the sample is optionally processed to extract, isolate and/or purify nucleic acid, in particular DNA or RNA, that may be present in the sample. Hence, in preferred embodiments, said sample on which a nucleic acid amplification reaction is performed is a processed biological or environmental sample as defined herein. Said processing is preferably to extract, isolate and/or purify nucleic acid. Extraction and/or isolation of nucleic acid is well known in the art and a skilled person is well capable of performing such extraction and/or isolation using e.g. commercially available nucleic acid, in particular DNA or RNA, extraction, isolation and/or purification kits.

The invention further provides a loop-mediated isothermal amplification (LAMP) primer set specific for *Legionella pneumophila* DotB gene, the primer set comprising a forward outer primer (F3), a backward outer primer (B3), a forward inner primer (FIP) and a backward inner primer (BIP), and optionally a loop forward primer (LoopF) and/or loop backward primer (LoopB), preferably at least one Loop primer, preferably a LoopF primer.

Also provided is a loop-mediated isothermal amplification (LAMP) primer set specific for *Legionella pneumophila* MIP gene, the primer set comprising a forward outer primer (F3), a backward outer primer (B3), a forward inner primer (FIP) and a backward inner primer (BIP), and optionally a loop forward primer (LoopF) and/or loop backward primer (LoopB), wherein:
- primer F3 comprises a consecutive stretch of at least 15 nucleotides of the sequence CAAAGTAATCAATTCTGGAAATGG;
- primer B3 comprises a consecutive stretch of at least 15 nucleotides of the sequence ACATAAATTTCCCAAGTTGATCC;
- primer FIP comprises a consecutive stretch of at least 30 nucleotides or two consecutive stretches of at least 30 nucleotides in total of the sequence GTACCATCAATCAGACGACCAGGTTAAACCCGGAAAATCGGA;
- primer BIP comprises a consecutive stretch of at least 30 nucleotides or two consecutive stretches of at least 30 nucleotides in total of the sequence CCGAAAAAACTGGTAAGCCAGCTGGCATCAATTGCAAAGC, and
   optionally
- primer LoopB comprises a consecutive stretch of at least 15 nucleotides of the sequence CAAGTTATCCCTGGATGGACAGAA.

As demonstrated in the examples herein, both a DotB LAMP primer set of the invention and a MIP LAMP primer set of the invention outperform previously known *Legionella* specific primer sets, in particular with respect to detection speed, detection limit and reactivity with a broad spectrum of *Legionella* isolates. Use of a combination of a DotB LAMP primer set of the invention and a MIP LAMP primer set of the invention provides the best results in terms of detection speed and taxonomic coverage.

The term "primer" refers to an oligonucleotide that is capable of acting as a point of initiation for the 5' to 3' synthesis of a primer extension product that is complementary to a nucleic acid strand. The primer extension product is synthesized in the presence of appropriate nucleotides and an agent for polymerization such as a DNA polymerase in an appropriate buffer and at a suitable temperature. Oligonucleotides useful as amplification primers in the present invention are typically DNA. The term "primer set" refers to two or more primers, in particular 4, 5 or 6 primers, more preferably 5 primers.

Where herein a sequence of a particular nucleic acid molecule is depicted, the sequence is depicted in the 5' to 3' orientation.

A "complementary" oligonucleotide, e.g. of a primer, corresponds to the antisense counterpart of a given oligonucleotide. That is, "A" is complementary to "T" and "G" to "C" and vice versa. Of course, this applies also to non-natural analogs of deoxynucleotides, as long as they are capable of "base-pairing" with their counterparts.

Primers of a primer set according to the invention or used in a method of the invention are specific for the DotB gene or the MIP gene of *Legionella pneumophila.* As used herein "specific for" means that the primers are capable of specifically hybridizing" to the nucleic acid of the indicated gene. The term "capable of specifically hybridizing to" refers to a nucleic acid sequence capable of specific base-pairing with a complementary nucleic acid sequence and binding thereto to form a nucleic acid duplex.

The terms "complementary" and "substantially complementary" refer to base pairing between nucleotides or nucleic acids, such as, for instance, between the two strands of a double-stranded DNA molecule or between an oligonucleotide primer and a primer binding site on a single- stranded nucleic acid to be sequenced or amplified. Complementary nucleotides are, generally, A and T (or A and U), and G and C. Within the context of the present invention, it is to be understood that the specific sequence lengths listed are illustrative and not limiting and that sequences covering the same map positions, but having slightly fewer or greater numbers of bases are deemed to be equivalents of the sequences and fall within the scope of the invention, provided they will hybridize to the same positions on the target as the listed sequences. Because it is understood that nucleic acids do not require complete complementarity in order to hybridize, the probe and primer sequences disclosed herein may be modified to some extent without loss of utility as specific primers and probes. Generally, sequences having homology of 90%, 95%, 96%, 97%, 98%, or 99% or more fall within the scope of the present invention. As is known in the art, hybridization of complementary and partially complementary nucleic acid sequences may be obtained by adjustment of the hybridization conditions to increase or decrease stringency, i.e., by adjustment of hybridization temperature or salt content of the buffer.

Primers for LAMP assays generally consist of 15-60 nucleotides, depending on the type of primer. Hence, in preferred embodiments, each primer of a primer set of the invention or used in a method of the invention is composed of 15-60 nucleotides.

The forward outer primer (F3) preferably comprises at least 15 nucleotides, more preferably 15-28 nucleotides, such as 15, 16, 17, 18, 19, 20, 21, 22, 23 or 24 nucleotides more preferably 18-24 nucleotides.

The backward outer primer (B3) preferably comprises at least 15 nucleotides, more preferably 15-28 nucleotides, such as 15, 16, 17, 18, 19, 20, 21, 22, 23 or 24 nucleotides more preferably 18-24 nucleotides.

The forward inner primer (FIP) preferably comprises at least 30 nucleotides, more preferably 30-60 nucleotides, more preferably 40-55 nucleotides, more preferably 40-50 nucleotides, such as 40, 41, 42, 43, 44, 45, 46, 47, 48, 49 or 50 nucleotides.

The backward inner primer (BIP) preferably comprises at least at least 30 nucleotides, more preferably 30-60 nucleotides, more preferably 40-55 nucleotides, more preferably 40-50 nucleotides, such as 40, 41, 42, 43, 44, 45, 46, 47, 48, 49 or 50 nucleotides.

The loop forward primer (LoopF), if present, preferably comprises at least 15 nucleotides, more preferably 15-24 nucleotides, such as 15, 16, 17, 18, 19, 20, 21, 22, 23 or 24 nucleotides more preferably 18-24 nucleotides.

The loop backward primer (LoopB), if present, preferably comprises at least 15 nucleotides, more preferably 15-24 nucleotides, such as 15, 16, 17, 18, 19, 20, 21, 22, 23 or 24 nucleotides more preferably 18-24 nucleotides.

In some embodiments, a primer set, preferably LAMP primer set, of the invention or used in a method of the invention is specific for the *Legionella pneumophila* DotB gene.

The DotB gene is part of the Dot/Icm system. The capacity of *Legionella* to infect eukaryotic cells relies on this system, which is a type IV secretion machinery that is translocating proteins into the eukaryotic host cell. The system has two major subcomplexes, the core transmembrane subcomplex and the coupling protein subcomplex. The core transmembrane subcomplex is composed of the proteins DotC, DotD, DotF (IcmG), DotG (IcmE), and DotH (IcmK) and crosses the inner and outer membranes of *L. pneumophila.* The coupling protein subcomplex, comprising DotL (IcmO), DotM (IcmP), DotN (IcmJ), a complex of two cytoplasmic chaperones, IcmS and IcmW (IcmSW) and the protein LvgA, recruits substrates and delivers them to the secretion channel.

DotB has not been previously reported as a target for molecular detection. This gene is highly conserved in *Legionella pneumophila* and was found to be exceptionally suitable for specific detection of *Legionella pneumophila.* As demonstrated in the Example, LAMP based detection assay using primers specific for the *Legionella pneumophila* DotB gene allows rapid and sensitive detection of *Legionella pneumophila.*

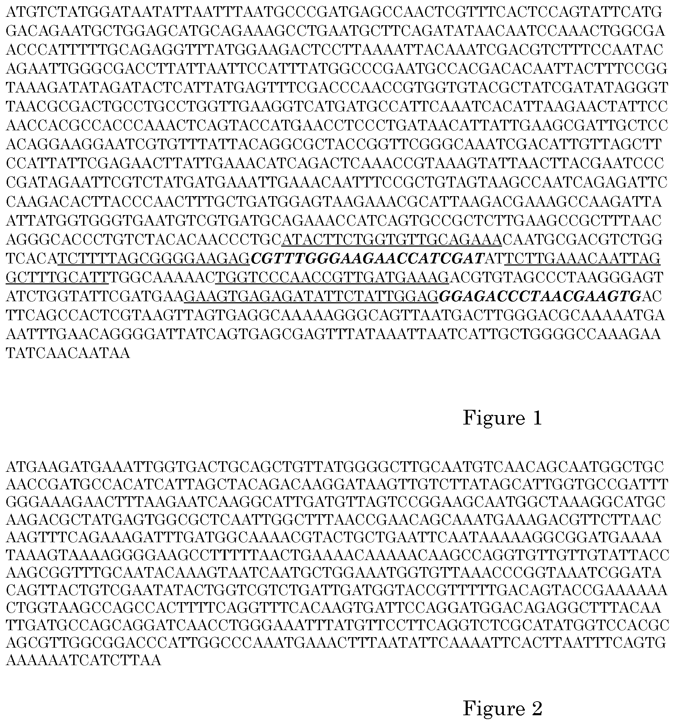

In preferred embodiments, detection of *Legionella pneumophila* based on the DotB gene is based on the sequence corresponding to nucleotides 3033061-3034194 of the sequence of Genbank accession number NZ_CP013742.1, which is the complete genome of *Legionella pneumophila* strain Philadelphia-1 isolate AE chromosome. These nucleotides 3033061-3034194 of this sequence are depicted in figure 1. Hence, preferably detection of *Legionella pneumophila* based on the DotB gene is preferably based on the sequence as depicted in figure 1. More preferably, it is based on a region of the DotB gene consisting of nucleotides 567-1134 as depicted in figure 1, preferably consisting of nucleotides 741-1034 as depicted in figure 1, more preferably consisting of nucleotides 769-1006 as depicted in figure 1, consisting of nucleotides 779-996 as depicted in figure 1.

Similarly, a primer set specific for the *Legionella pneumophila* DotB gene of the invention or used in a method of the invention is preferably specific for the sequence as depicted in figure 1, more preferably specific for a region of the DotB gene consisting of nucleotides 567-1134 as depicted in figure 1, preferably consisting of nucleotides 741-1034 as depicted in figure 1, more preferably consisting of nucleotides 769-1006 as depicted in figure 1, consisting of nucleotides 779-996 as depicted in figure 1.

The skilled person will know how to design suitable (LAMP) primers specific for the DotB gene.

A LAMP primer set specific for the *Legionella pneumophila* DotB gene of the invention or used in a method of the invention preferably comprises the following primers: a forward outer primer (F3), a backward outer primer (B3), a forward inner primer (FIP) and a backward inner primer (BIP). The primer set may optionally further comprise a loop forward primer (LoopF) and/or loop backward primer (LoopB). In preferred embodiments the primer set comprises at least a LoopF primer. In further preferred embodiments, the primer set consists of 5 primers: the F3, B3, FIP, BIP and LoopF primers.

In preferred embodiments, the F3 primer comprises a consecutive stretch of at least 15 nucleotides of the sequence ATACTTCTGGTGTTGCAGAAA, more preferably of at least 16, more preferably of at least 17, more preferably of at least 18, more preferably of at least 19, more preferably of at least 20, more preferably of at least 21 nucleotides of said sequence. Preferably the primer has a maximum length of 28 nucleotides, more preferably 24 nucleotides, more preferably 21 nucleotides.

In preferred embodiments, the B3 primer comprises a consecutive stretch of at least 15 nucleotides of the sequence CACTTCGTTAGGGTCTCC, more preferably of at least 16, more preferably of at least 17, more preferably of at least 18 nucleotides of said sequence. Preferably the primer has a maximum length of 28 nucleotides, more preferably 24 nucleotides, more preferably 21 nucleotides, more preferably 18 nucleotides.

In preferred embodiments, the FIP primer comprises a consecutive stretch of at least 30 nucleotides or two consecutive stretches of at least 30 nucleotides in total of the sequence AATGCAAAGCCTAATTGTTTCAAGATCTTTTAGCGGGGAAGAG, more preferably of at least 35, more preferably of at least 38, more preferably of at least 39, more preferably of at least 40, more preferably of at least 41, more preferably at of least 42, more preferably of at least 43 nucleotides of said sequence. Preferably the primer has a maximum length of 60 nucleotides, more preferably 55 nucleotides, more preferably 50 nucleotides, more preferably 43 nucleotides. The two consecutive stretches of at least 30 nucleotides in total are optionally separated by up to 10 nucleotides, preferably up to 8 nucleotides, preferably up to 6 nucleotides, preferably up to 5 nucleotides. E.g. the two consecutive stretches of at least 30 nucleotides in total are separated poly-T linker, of up to 10, preferably up to 8, preferably up to 6, preferably up to 5 thymine nucleotides. In further preferred embodiments, the FIP primer comprises a consecutive stretch of at least 18 nucleotides of the sequence AATGCAAAGCCTAATTGTTTCAAGA and a consecutive stretch of at least 12 nucleotides of the sequence TGGCATCAATTGCAAAGC, which two consecutive stretches of nucleotides are optionally separated by up to 10 nucleotides, preferably up to 8 nucleotides, preferably up to 6 nucleotides, preferably up to 5 nucleotides. More preferably, FIP primer comprises a consecutive stretch of at least 20, preferably at least 22, preferably at least 23, preferably at least 24, more preferably all 25, nucleotides of the sequence AATGCAAAGCCTAATTGTTTCAAGA and a consecutive stretch of at least 14, preferably at least 15, preferably at least 16, preferably at least 17, more preferably all 18, nucleotides of the sequence TGGCATCAATTGCAAAGC, which two consecutive stretches of nucleotides are optionally separated by up to 10 nucleotides, preferably up to 8 nucleotides, preferably up to 6 nucleotides, preferably up to 5 nucleotides.

In preferred embodiments, the BIP primer comprises a consecutive stretch of at least 30 nucleotides or two consecutive stretches of at least 30 nucleotides in total of the sequence TGGTCCCAACCGTTGATGAAAGCTCCAATAGAATATCTCTCACTTC, more preferably of at least 35, more preferably of at least 38, more preferably of at least 40, more preferably of at least 41, more preferably of at least 42, more preferably of at least 43 more preferably of at least 44, more preferably of at least 45, more preferably of at least 46 nucleotides of said sequence. Preferably the primer has a maximum length of 60 nucleotides, more preferably 55 nucleotides, more preferably 50 nucleotides, more preferably 46 nucleotides. The two consecutive stretches of at least 30 nucleotides in total are optionally separated by up to 10 nucleotides, preferably up to 8 nucleotides, preferably up to 6 nucleotides, preferably up to 5 nucleotides. E.g. the two consecutive stretches of at least 30 nucleotides in total are separated poly-T linker, of up to 10, preferably up to 8, preferably up to 6, preferably up to 5 thymine nucleotides. In further preferred embodiments, the BIP primer comprises a consecutive stretch of at least 14 nucleotides of the sequence TGGTCCCAACCGTTGATGAAAG and a consecutive stretch of at least 16 nucleotides of the sequence CTCCAATAGAATATCTCTCACTTC, which two consecutive stretches of nucleotides are optionally separated by up to 10 nucleotides, preferably up to 8 nucleotides, preferably up to 6 nucleotides, preferably up to 5 nucleotides. More preferably, the BIP primer comprises a consecutive stretch of at least 16, preferably at least 18, preferably at least 20, preferably at least 21, more preferably all 22, nucleotides of the sequence TGGTCCCAACCGTTGATGAAAG and a consecutive stretch of at least 18, preferably at least 20, preferably at least 22, preferably at least 24, more preferably all 24, nucleotides of the sequence CTCCAATAGAATATCTCTCACTTC, which two consecutive stretches of nucleotides are optionally separated by up to 10 nucleotides, preferably up to 8 nucleotides, preferably up to 6 nucleotides, preferably up to 5 nucleotides.

In preferred embodiments, if present, the LoopF primer comprises a consecutive stretch of at least 15 nucleotides of the sequence ATCGATGGTTCTTCCCAAACG, more preferably at least 16, more preferably at least 17, more preferably at least 18, more preferably at least 19, more preferably at least 20, more preferably at least 21 nucleotides of said sequence. Preferably the primer has a maximum length of 28 nucleotides, more preferably 24 nucleotides, more preferably 21 nucleotides.

In preferred embodiments, the F3 primer of the LAMP primer set specific for the *Legionella pneumophila* DotB gene comprises or consists of a consecutive stretch of 15, 16, 17, 18, 19, 20 or 21 nucleotides of the sequence ATACTTCTGGTGTTGCAGAAA. In further preferred embodiments, the F3 primer comprises or consists of a consecutive stretch of 18, 19, 20 or 21 nucleotides of the sequence ATACTTCTGGTGTTGCAGAAA. In further preferred embodiments, the F3 primer comprises or consists of a consecutive stretch of 20 or 21 nucleotides of the sequence ATACTTCTGGTGTTGCAGAAA. In further preferred embodiments, primer F3 comprises or consists of the sequence ATACTTCTGGTGTTGCAGAAA.

In preferred embodiments, the B3 primer of the LAMP primer set specific for the *Legionella pneumophila* DotB gene comprises or consists of a consecutive stretch of 15, 16, 17 or 18 nucleotides of the sequence CACTTCGTTAGGGTCTCC. In further preferred embodiments, the B3 primer comprises or consists of a consecutive stretch of 17 or 18, nucleotides of the sequence CACTTCGTTAGGGTCTCC. In further preferred embodiments, primer B3 comprises or consists of the sequence CACTTCGTTAGGGTCTCC.

In preferred embodiments, the FIP primer of the LAMP primer set specific for the *Legionella pneumophila* DotB gene comprises a consecutive stretch of at least 35 nucleotides or two consecutive stretches of at least 35 nucleotides in total of the sequence AATGCAAAGCCTAATTGTTTCAAGATCTTTTAGCGGGGAAGAG. In further preferred embodiments, the FIP primer comprises or consists of a consecutive stretch of 30, 31, 32, 33, 34, 35, 36, 37, 38, 39, 40, 41, 42 or 43 nucleotides or two consecutive stretches of 30, 31, 32, 33, 34, 35, 36, 37, 38, 39, 40, 41, 42 or 43 nucleotides in total of the sequence AATGCAAAGCCTAATTGTTTCAAGATCTTTTAGCGGGGAAGAG. In further preferred embodiments, the FIP primer comprises or consists of a consecutive stretch of 30, 31, 32, 33, 34, 35, 36, 37, 38, 39, 40, 41, 42 or 43 nucleotides of the sequence AATGCAAAGCCTAATTGTTTCAAGATCTTTTAGCGGGGAAGAG. In further preferred embodiments, the FIP primer comprises or consists of a consecutive stretch of 41, 42 or 43 nucleotides of the sequence AATGCAAAGCCTAATTGTTTCAAGATCTTTTAGCGGGGAAGAG. In further preferred embodiments, primer FIP comprises or consists of the sequence AATGCAAAGCCTAATTGTTTCAAGATCTTTTAGCGGGGAAGAG.

In preferred embodiments, the BIP primer of the LAMP primer set specific for the *Legionella pneumophila* DotB gene comprises a consecutive stretch of at least 35 nucleotides or two consecutive stretches of at least 35 nucleotides in total of the sequence TGGTCCCAACCGTTGATGAAAGCTCCAATAGAATATCTCTCACTTC. In further preferred embodiments, the BIP primer comprises or consists of a consecutive stretch of 30, 31, 32, 33, 34, 35, 36, 37, 38, 39, 40, 41, 42, 43, 44, 45 or 46 nucleotides of the sequence TGGTCCCAACCGTTGATGAAAGCTCCAATAGAATATCTCTCACTTC. In further preferred embodiments, the BIP primer comprises or consists of a consecutive stretch of 30, 31, 32, 33, 34, 35, 36, 37, 38, 39, 40, 41, 42, 43, 44, 45 or 46 nucleotides or two consecutive stretches of 30, 31, 32, 33, 34, 35, 36, 37, 38, 39, 40, 41, 42, 43, 44, 45 or 46 nucleotides in total of the sequence TGGTCCCAACCGTTGATGAAAGCTCCAATAGAATATCTCTCACTTC. In further preferred embodiments, the BIP primer comprises or consists of a consecutive stretch of 43, 44, 45 or 46 nucleotides of the sequence TGGTCCCAACCGTTGATGAAAGCTCCAATAGAATATCTCTCACTTC. In further preferred embodiments, primer BIP comprises or consists of the sequence TGGTCCCAACCGTTGATGAAAGCTCCAATAGAATATCTCTCACTTC.

In preferred embodiments, the LoopF primer of the LAMP primer set specific for the *Legionella pneumophila* DotB gene comprises or consists of a consecutive stretch of 15, 16, 17, 18, 19, 20 or 21 nucleotides of the sequence ATCGATGGTTCTTCCCAAACG. In further preferred embodiments, the LoopF primer comprises or consists of a consecutive stretch of 18, 19, 20 or 21 nucleotides of the sequence ATCGATGGTTCTTCCCAAACG. In further preferred embodiments, the LoopF primer comprises or consists of a consecutive stretch of 20 or 21 nucleotides of the sequence ATCGATGGTTCTTCCCAAACG. In further preferred embodiments, primer LoopF comprises or consists of the sequence ATCGATGGTTCTTCCCAAACG.

Preferred embodiments for each of the DotB gene specific primers are further preferably combined in a primer set of the invention or used in a method of the invention. For example, in preferred embodiments:
- primer F3 comprises a consecutive stretch of at least 15 nucleotides of the sequence ATACTTCTGGTGTTGCAGAAA;
- primer B3 comprises a consecutive stretch of at least 15 nucleotides of the sequence CACTTCGTTAGGGTCTCC;
- primer FIP comprises a consecutive stretch of at least 30 nucleotides or two consecutive stretches of at least 30 nucleotides in total of the sequence AATGCAAAGCCTAATTGTTTCAAGATCTTTTAGCGGGGAAGAG;
- primer BIP comprises a consecutive stretch of at least 30 nucleotides or two consecutive stretches of at least 30 nucleotides in total of the sequence TGGTCCCAACCGTTGATGAAAGCTCCAATAGAATATCTCTCACTTC, and
- primer LoopF comprises a consecutive stretch of at least 15 nucleotides of the sequence ATCGATGGTTCTTCCCAAACG.

As another example, in further preferred embodiments:
- primer F3 comprises or consists of the sequence ATACTTCTGGTGTTGCAGAAA;
- primer B3 comprises or consists of the sequence CACTTCGTTAGGGTCTCC;
- primer FIP comprises or consists of the sequence AATGCAAAGCCTAATTGTTTCAAGATCTTTTAGCGGGGAAGAG;
- primer BIP comprises or consists of the sequence TGGTCCCAACCGTTGATGAAAGCTCCAATAGAATATCTCTCACTTC, and
- primer LoopF comprises or consists of the sequence ATCGATGGTTCTTCCCAAACG.

It is further preferred that the sequences of the primers specific for the *Legionella pneumophila* DotB gene are 100% identical to the sequence of the primers exemplified herein.

In some embodiments, a primer set of the invention or used in a method of the invention is specific for the *Legionella pneumophila* MIP gene, preferably for the sequence of the MIP gene as depicted in figure 2.

The *Legionella* macrophage infectivity potentiator (MIP) is a virulence factor that is responsible for the intracellular survival of *Legionella* species. The MIP gene can be used to distinguish different species of *Legionella* and detection based on MIP gene is thus used to identify *Legionella pneumophila.*

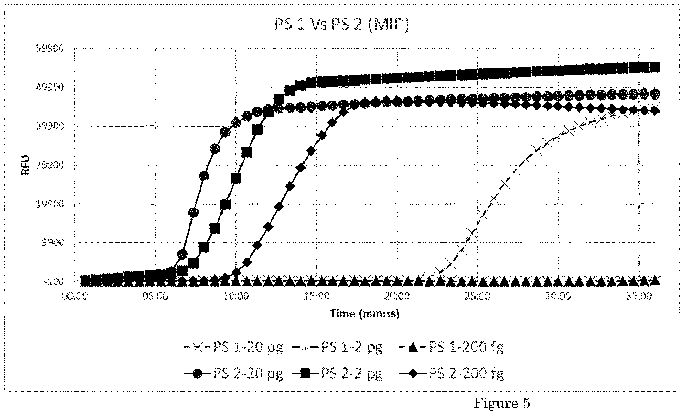

Several PCT based methods and a LAMP based method based on the detection of the *Legionella pneumophila* . However, the present inventors have identified LAMP primers specific for the MIP gene that outperform the currently known LAMP primer set as demonstrated in the Examples herein and shown in figure 5. In particular the specific LAMP primers of the present invention were found to result in a more rapid and sensitive LAMP assay as compared to known LAMP primers specific for the MIP gene.

A LAMP primer set specific for the *Legionella pneumophila* MIP gene of the invention or used in a method of the invention preferably comprises the following primers: a forward outer primer (F3), a backward outer primer (B3), a forward inner primer (FIP) and a backward inner primer (BIP). The primer set may optionally further comprise a loop forward primer (LoopF) and/or loop backward primer (LoopB). In preferred embodiments the primer set comprises at least one Loop primer, preferably a LoopB primer. In further preferred embodiments, the primer set consists of 5 primers: the F3, B3, FIP, BIP and LoopB primers.

In preferred embodiments, the F3 primer comprises a consecutive stretch of at least 15 nucleotides of the sequence CAAAGTAATCAATTCTGGAAATGG, more preferably of at least 18, more preferably of at least 20, more preferably of at least 21, more preferably of at least 22, more preferably of at least 23, more preferably of at least 24 nucleotides of said sequence. Preferably the primer has a maximum length of 32 nucleotides, more preferably 28 nucleotides, more preferably 24 nucleotides.

In preferred embodiments, the B3 primer comprises a consecutive stretch of at least 15 nucleotides of the sequence ACATAAATTTCCCAAGTTGATCC, more preferably of at least 18, more preferably of at least 20, more preferably of at least 21, more preferably of at least 22, more preferably of at least 23 nucleotides of said sequence. Preferably the primer has a maximum length of 30 nucleotides, more preferably 27 nucleotides, more preferably 23 nucleotides.

In preferred embodiments, the FIP primer comprises a consecutive stretch of at least 30 nucleotides or two consecutive stretches of at least 30 nucleotides in total of the sequence GTACCATCAATCAGACGACCAGGTTAAACCCGGAAAATCGGA, more preferably of at least 33, more preferably of at least 35, more preferably of at least 37, more preferably of at least 38, more preferably of at least 39 more preferably of at least 40, more preferably of at least 41, more preferably of at least 42 nucleotides of said sequence. Preferably the primer has a maximum length of 60 nucleotides, more preferably 55 nucleotides, more preferably 55 nucleotides more preferably 42 nucleotides. The two consecutive stretches of at least 30 nucleotides in total are optionally separated by up to 10 nucleotides, preferably up to 8 nucleotides, preferably up to 6 nucleotides, preferably up to 5 nucleotides. E.g. the two consecutive stretches of at least 30 nucleotides in total are separated poly-T linker, of up to 10, preferably up to 8, preferably up to 6, preferably up to 5 thymine nucleotides. In further preferred embodiments, the FIP primer comprises a consecutive stretch of at least 15 nucleotides of the sequence GTACCATCAATCAGACGACCAG and a consecutive stretch of at least 15 nucleotides of the sequence GTTAAACCCGGAAAATCGGA, which two consecutive stretches of nucleotides are optionally separated by up to 10 nucleotides, preferably up to 8 nucleotides, preferably up to 6 nucleotides, preferably up to 5 nucleotides. More preferably, FIP primer comprises a consecutive stretch of at least 16, preferably at least 18, preferably at least 20, preferably at least 21, more preferably all 22, nucleotides of the sequence GTACCATCAATCAGACGACCAG and a consecutive stretch of at least 16, preferably at least 17, preferably at least 18, preferably at least 19, more preferably all 20, nucleotides of the sequence GTTAAACCCGGAAAATCGGA, which two consecutive stretches of nucleotides are optionally separated by up to 10 nucleotides, preferably up to 8 nucleotides, preferably up to 6 nucleotides, preferably up to 5 nucleotides.

In preferred embodiments, the BIP primer comprises a consecutive stretch of at least 30 nucleotides or two consecutive stretches of at least 30 nucleotides in total of the sequence CCGAAAAAACTGGTAAGCCAGCTGGCATCAATTGCAAAGC, more preferably of at least 33, more preferably of at least 35, more preferably of at least 36, more preferably of at least 37, more preferably of at least 38, more preferably of at least 39 more preferably of at least 40 nucleotides of said sequence. Preferably the primer has a maximum length of 60 nucleotides, more preferably 55 nucleotides, more preferably 50 nucleotides, more preferably 45 nucleotides, more preferably 40 nucleotides. The two consecutive stretches of at least 30 nucleotides in total are optionally separated by up to 10 nucleotides, preferably up to 8 nucleotides, preferably up to 6 nucleotides, preferably up to 5 nucleotides. E.g. the two consecutive stretches of at least 30 nucleotides in total are separated poly-T linker, of up to 10, preferably up to 8, preferably up to 6, preferably up to 5 thymine nucleotides. In further preferred embodiments, the BIP primer comprises a consecutive stretch of at least 16 nucleotides of the sequence CCGAAAAAACTGGTAAGCCAGC and a consecutive stretch of at least 14 nucleotides of the sequence TGGCATCAATTGCAAAGC, which two consecutive stretches of nucleotides are optionally separated by up to 10 nucleotides, preferably up to 8 nucleotides, preferably up to 6 nucleotides, preferably up to 5 nucleotides. More preferably, the BIP primer comprises a consecutive stretch of at least 18, preferably at least 19, preferably at least 20, preferably at least 21, more preferably all 22, nucleotides of the sequence CCGAAAAAACTGGTAAGCCAGC and a consecutive stretch of at least 15 preferably at least 16, preferably at least 17, more preferably all 18, nucleotides of the sequence TGGCATCAATTGCAAAGC, which two consecutive stretches of nucleotides are optionally separated by up to 10 nucleotides, preferably up to 8 nucleotides, preferably up to 6 nucleotides, preferably up to 5 nucleotides.

In preferred embodiments, if present, the LoopB primer comprises a consecutive stretch of at least 15 nucleotides of the sequence CAAGTTATCCCTGGATGGACAGAA, more preferably of at least 18, more preferably of at least 20, more preferably of at least 21, more preferably of at least 22, more preferably of at least 23, more preferably of at least 24 nucleotides of said sequence. Preferably the primer has a maximum length of 32 nucleotides, more preferably 28 nucleotides, more preferably 24 nucleotides.

In preferred embodiments, the F3 primer of the LAMP primer set specific for the *Legionella pneumophila* MIP gene comprises or consists of a consecutive stretch of 15, 16, 17, 18, 19, 20, 21, 22, 23 or 24 nucleotides of the sequence CAAAGTAATCAATTCTGGAAATGG. In further preferred embodiments, the F3 primer comprises or consists of a consecutive stretch of 18, 19, 20, 21, 22, 23 or 24 nucleotides of the sequence CAAAGTAATCAATTCTGGAAATGG. In further preferred embodiments, the F3 primer comprises or consists of a consecutive stretch of 21, 22, 23 or 24 nucleotides of the sequence CAAAGTAATCAATTCTGGAAATGG. In further preferred embodiments, the F3 primer comprises or consists of a consecutive stretch of 23 or 24 nucleotides of the sequence CAAAGTAATCAATTCTGGAAATGG. In further preferred embodiments, primer F3 comprises or consists of the sequence CAAAGTAATCAATTCTGGAAATGG.

In preferred embodiments, the B3 primer of the LAMP primer set specific for the *Legionella pneumophila* MIP gene comprises or consists of a consecutive stretch of 15, 16, 17, 18, 19, 20, 21, 22 or 23 nucleotides of the sequence ACATAAATTTCCCAAGTTGATCC. In further preferred embodiments, the B3 primer comprises or consists of a consecutive stretch of 18, 19, 20, 21, 22 or 23 nucleotides of the sequence ACATAAATTTCCCAAGTTGATCC. In further preferred embodiments, the B3 primer comprises or consists of a consecutive stretch of 20, 21, 22 or 23 nucleotides of the sequence ACATAAATTTCCCAAGTTGATCC. In further preferred embodiments, the B3 primer comprises or consists of a consecutive stretch of 22 or 23 nucleotides of the sequence ACATAAATTTCCCAAGTTGATCC. In further preferred embodiments, primer B3 comprises or consists of the sequence ACATAAATTTCCCAAGTTGATCC.

In preferred embodiments, the FIP primer of the LAMP primer set specific for the *Legionella pneumophila* MIP gene comprises a consecutive stretch of 30, 31, 32, 33, 34, 35, 36, 37, 38, 39, 40, 41 or 42 nucleotides or two consecutive stretches of 30, 31, 32, 33, 34, 35, 36, 37, 38, 39, 40, 41, or 42 nucleotides in total of the sequence GTACCATCAATCAGACGACCAGGTTAAACCCGGAAAATCGGA. In preferred embodiments, the FIP primer of the LAMP primer set specific for the *Legionella pneumophila* MIP gene comprises a consecutive stretch of 30, 31, 32, 33, 34, 35, 36, 37, 38, 39, 40, 41 or 42 nucleotides of the sequence GTACCATCAATCAGACGACCAGGTTAAACCCGGAAAATCGGA. In further preferred embodiments, the FIP primer comprises or consists of a consecutive stretch of 39, 40, 41 or 42 nucleotides of the sequence GTACCATCAATCAGACGACCAGGTTAAACCCGGAAAATCGGA. In further preferred embodiments, the FIP primer comprises or consists of a consecutive stretch of 41 or 42 nucleotides of the sequence GTACCATCAATCAGACGACCAGGTTAAACCCGGAAAATCGGA. In further preferred embodiments, primer FIP comprises or consists of the sequence GTACCATCAATCAGACGACCAGGTTAAACCCGGAAAATCGGA.

In preferred embodiments, the BIP primer of the LAMP primer set specific for the *Legionella pneumophila* MIP gene comprises a consecutive stretch 30, 31, 32, 33, 34, 35, 36, 37, 38, 39 or 40 nucleotides or two consecutive stretches of 30, 31, 32, 33, 34, 35, 36, 37, 38, 39 or 40 nucleotides in total of the sequence CCGAAAAAACTGGTAAGCCAGCTGGCATCAATTGCAAAGC. In preferred embodiments, the BIP primer of the LAMP primer set specific for the *Legionella pneumophila* MIP gene comprises a consecutive stretch 30, 31, 32, 33, 34, 35, 36, 37, 38, 39 or 40 nucleotides of the sequence CCGAAAAAACTGGTAAGCCAGCTGGCATCAATTGCAAAGC. In further preferred embodiments, the BIP primer comprises or consists of a consecutive stretch of 37, 38, 39 or 40 nucleotides of the sequence CCGAAAAAACTGGTAAGCCAGCTGGCATCAATTGCAAAGC. In further preferred embodiments, the BIP primer comprises or consists of a consecutive stretch of 39 or 40 nucleotides of the sequence CCGAAAAAACTGGTAAGCCAGCTGGCATCAATTGCAAAGC. In further preferred embodiments, primer BIP comprises or consists of the sequence CCGAAAAAACTGGTAAGCCAGCTGGCATCAATTGCAAAGC.

In preferred embodiments, the LoopB primer of the LAMP primer set specific for the *Legionella pneumophila* MIP gene comprises or consists of a consecutive stretch of 15, 16, 17, 18, 19, 20, 21, 22, 23 or 24 nucleotides of the sequence CAAGTTATCCCTGGATGGACAGAA. In further preferred embodiments, the LoopB primer comprises or consists of a consecutive stretch of 18, 19, 20, 21, 22, 23 or 24 nucleotides of the sequence CAAGTTATCCCTGGATGGACAGAA. In further preferred embodiments, the LoopB primer comprises or consists of a consecutive stretch of 21, 22, 23 or 24 nucleotides of the sequence CAAGTTATCCCTGGATGGACAGAA. In further preferred embodiments, the LoopB primer comprises or consists of a consecutive stretch of 23 or 24 nucleotides of the sequence CAAGTTATCCCTGGATGGACAGAA. In further preferred embodiments, primer LoopB comprises or consists of the sequence CAAGTTATCCCTGGATGGACAGAA.

Preferred embodiments for each of the MIP gene specific primers are further preferably combined in a primer set of the invention or used in a method of the invention. For example, in preferred embodiments:
- primer F3 comprises a consecutive stretch of at least 15 nucleotides of the sequence CAAAGTAATCAATTCTGGAAATGG;
- primer B3 comprises a consecutive stretch of at least 15 nucleotides of the sequence ACATAAATTTCCCAAGTTGATCC;
- primer FIP comprises a consecutive stretch of at least 30 nucleotides or two consecutive stretches of at least 30 nucleotides in total of the sequence GTACCATCAATCAGACGACCAGGTTAAACCCGGAAAATCGGA;
- primer BIP comprises a consecutive stretch of at least 30 nucleotides or two consecutive stretches of at least 30 nucleotides in total of the sequence CCGAAAAAACTGGTAAGCCAGCTGGCATCAATTGCAAAGC, and
- primer LoopB comprises a consecutive stretch of at least 15 nucleotides of the sequence CAAGTTATCCCTGGATGGACAGAA.

As another example, in further preferred embodiments:
- primer F3 comprises or consists of the sequence CAAAGTAATCAATTCTGGAAATGG;
- primer B3 comprises or consists of the sequence ACATAAATTTCCCAAGTTGATCC;
- primer FIP comprises or consists of the sequence GTACCATCAATCAGACGACCAGGTTAAACCCGGAAAATCGGA;
- primer BIP comprises or consists of the sequence CCGAAAAAACTGGTAAGCCAGCTGGCATCAATTGCAAAGC, and
- primer LoopB comprises or consists of the sequence CAAGTTATCCCTGGATGGACAGAA.

It is further preferred that the sequences of the primers specific for the *Legionella pneumophila* MIP gene are 100% identical to the sequence of the primers exemplified herein.

In some preferred embodiments, a MIP primer set according to the invention and a DotB primer set according to the invention are combined. Preferably, both a MIP primer set according to the invention and a DotB primer set according to the invention are included in a kit of the invention or used in a method of the invention.

Amplification involves the use of a polymerase enzyme. Any polymerase can be used for amplification of *Legionella* nucleic acid. The skilled person knows what polymerases are suitable to conduct an iNAAT, preferably LAMP, reaction. In preferred embodiments, the DNA polymerase is a polymerase from *Bacillus stearothermophilus* (Bst) or a homologue thereof, also referred to as a Bst DNA polymerase, such as Bst long fragment (Bst-Lf), Bst2.0 DNA polymerase, , or DNA polymerase I of *Parageobacillus yumthangensis* or a homologue thereof, such as the long fragment thereof. A particularly preferred polymerase is a Bst DNA polymerase.

In further preferred embodiments, the iNAAT reaction, in particular LAMP reaction, is performed in reaction buffer comprising one or more components selected from guanidine, ammonium sulphate, polysorbate (in particular tween), preferably at least two of said components, preferably all three. It is further preferred that at least guanidine and ammonium sulphate are present in the reaction buffer. These components contribute to the speed and sensitivity of the iNAAT, in particular LAMP, reaction. If present, guanidine is preferably present in the reaction buffer in a concentration of 20-100 mM, more preferably from 25-60 mM, more preferably from 30-50 mM, e.g. 35-45 mM or about 40 mM. If present, ammonium sulphate is preferably present in the reaction buffer in a concentration of 15-100 mM, more preferably from 20-50 mM, more preferably from 20-40 mM, e.g. 25-30 mM. If present, tween is preferably present in the reaction buffer in a concentration of 0.05-1 wt% of Tween20, or a corresponding concentration of another tween product, more preferably 0.05-0.5 % v/v, more preferably 0.06 - 0.2 % v/v, e.g. 0.075-0.125 % v/v or about 0.1 % v/v. As used herein a corresponding concentration of another tween product refers to a concentration for the product that provides the same properties as the indicated concentration of Tween20.

The term "hybridizing conditions" is intended to mean those conditions of time, temperature, and pH, and the necessary amounts and concentrations of reactants and reagents, sufficient to allow at least a portion of complementary sequences to anneal with each other. As is well known in the art, the time, temperature, and pH conditions required to accomplish hybridization depend on the size of the oligonucleotide probe or primer to be hybridized, the degree of complementarity between the oligonucleotide primer and the target, and the presence of other materials in the hybridization reaction admixture. The actual conditions necessary for each hybridization step are well known in the art or can be determined without undue experimentation.

The term "label" as used herein refers to any atom or molecule that can be used to provide a detectable (preferably quantifiable) signal, and that can be attached to a nucleic acid via a covalent bond or noncovalent interaction (e.g., through ionic or hydrogen bonding, or via immobilization, adsorption, or the like). Labels generally provide signals detectable by fluorescence, chemiluminescence, radioactivity, colorimetry, mass spectrometry, X-ray diffraction or absorption, magnetism, enzymatic activity, or the like. Examples of labels include fluorophores, chromophores, radioactive atoms, electron- dense reagents, enzymes, and ligands having specific binding partners. In one embodiment, a label does not comprise nucleotides, i.e. said label is a non-nucleotide label.

The methods of the invention comprise performing an isothermal nucleic acid amplification reaction. Said reaction is in particular for the specific amplification of *Legionella pneumophila* nucleic acid, in particular specific amplification of *Legionella pneumophila* DotB gene nucleic acid or *Legionella pneumophila* MIP gene nucleic acid. Performing an amplification reaction generally includes contacting the sample, preferably processed sample to extract, isolate and or purify nucleic acid as described herein, with DotB specific primers or the MIP specific primers of the invention to produce a *Legionella pneumophila* amplification product if *Legionella pneumophila* is present in the sample. "Amplification product" in the context of the present invention refers to the nucleic acid that is the result of the amplification reaction. This typically contains the DNA sequence of the region of *Legionella pneumophila* that is spanned by the primer set used for the amplification flanked by the sequence of the outer primers of the set used for the amplification, such as the F3 and B3 primers in a LAMP reaction. In preferred embodiments, the step of performing an amplification reaction comprises incubating a sample, preferably processed sample to extract, isolate and or purify nucleic acid as described herein, with a primer set specific for the *Legionella pneumophila* DotB gene as described herein or with a primer set specific for the *Legionella pneumophila* MIP gene according to the invention.

The amplification step of a method of the invention is performed at a constant temperature, preferably of 60-70 °C, such as at 65°C or 67°C. In preferred embodiments, the amplification step of a method of the invention is performed at a temperature of about 67°C. In preferred embodiments, a method of the invention is performed at a temperature of about 67°C.

In some embodiments, a method of the invention comprises a reverse transcription reaction, wherein *Legionalla pneumophila* RNA is transcribed into cDNA. In some embodiments, a method of the invention comprises performing an RT-LAMP reaction. Hence, in some embodiments the isothermal nucleic acid amplification (iNAAT) reaction performed in a method of the invention is an RT-LAMP reaction.

A method of the invention further preferably comprises determining whether the sample comprises an amplification product of the amplification reaction. In preferred embodiments this comprises detecting the presence or absence of amplification product. Such detection can be performed with any method known in the art for detection of amplification product of an iNAAT, preferably LAMP, reaction, including but not limited to the use of fluorescent intercalating dyes, fluorescent primers or probes, measuring turbidity, electrochemical probes, bioluminescent signals, chemiluminescent probes, changes in pH and magnesium based dyes.

In some embodiments detecting the presence or absence of amplification product comprises detecting the presence or absence of binding of a double-stranded DNA binding dye or intercalating dye into the amplification product. A method of the invention may therefore comprise a dye-binding step. A dye-binding step generally includes contacting the amplification products with a double-stranded DNA binding or intercalating dye. The presence of binding is typically indicative of the presence of *Legionella pneumophila* amplification product and thus of *Legionella pneumophila* nucleic acid in the sample. Hence, amplification product is detected if binding is detected. The absence of binding is typically indicative of the absence of *Legionella pneumophila* amplification product and thus of nucleic acid in the sample. Hence, amplification product is not detected if binding is detected. A method of the invention may further include the step of determining the melting temperature between the amplification product and the double-stranded DNA binding dye. Generally, the melting temperature confirms the presence or absence of *Legionella pneumophila* amplification product and thus of *Legionella pneumophila* nucleic acid in the sample. Suitable double-stranded DNA binding or intercalating dyes include SYTO-9^{®}, SYTO-13^{®}, SYTO-82^{®}, SYBR Green I^{®}, SYBR Gold^{®} and EvaGreen^{®}.

In some embodiments detecting the presence or absence of amplification product comprises measuring turbidity in the reaction mixture during the amplification reaction. Turbidity measurement is based on turbidity derived from magnesium pyrophosphate formation in the reaction mixture. The LAMP method allows to synthesize large amounts of DNA. Consequently, a large amount of pyrophosphate ion, a by-product of DNA synthesis, is produced. This yields white precipitate of magnesium pyrophosphate in the reaction mixture. Measuring the presence or absence of this precipitate allows detecting the presence or absence of amplified nucleic acid by the LAMP reaction. And because an increase in the turbidity of the reaction mixture according to the production of precipitate correlates with the amount of DNA amplified, real-time monitoring of the LAMP reaction is possible by real-time measurement of turbidity.

In some preferred embodiments, the step of determining whether the sample comprises an amplification product comprises monitoring amplification of the during the amplification process, i.e. real-time detection and/or monitoring of the amplification reaction. Real-time detection and monitoring is possible for both detection based on double-stranded DNA binding or intercalating dyes or on turbidity measurements.

The invention further provides a kit of parts comprising a LAMP primer set according to the invention and a DNA polymerase, nucleotides and/or a reaction buffer.

Nucleotides are for instance commercially available as a dNTP mix.

In preferred embodiments, the DNA polymerase in a kit of parts of the invention is a polymerase from *Bacillus stearothermophilus* (Bst) or a homologue thereof, also referred to as a Bst DNA polymerase, such as Bst long fragment (Bst-Lf), a Bst2.0 DNA polymerase, or DNA polymerase I of *Parageobacillus yumthangensis* or a homologue thereof, such as the long fragment thereof. In further preferred embodiments, reaction buffer present in a kit of parts of the invention comprises one or more components selected from guanidine, ammonium sulphate, tween, preferably at least two of said components, preferably all three. It is further preferred that at least guanidine and ammonium sulphate are present in the reaction buffer. If present, guanidine is preferably present in the reaction buffer in a concentration of 20-100 mM, more preferably from 25-60 mM, more preferably from 30-50 mM, e.g. 35-45 mM or about 40 mM. If present, ammonium sulphate is preferably present in the reaction buffer in a concentration of 15-100 mM, more preferably from 20-50 mM, more preferably from 20-40 mM, e.g. 25-30 mM. If present, tween is preferably present in the reaction buffer in a concentration of 0.05-1 wt% of Tween20, or a corresponding concentration of another tween product, more preferably 0.05-0.5 % v/v, more preferably 0.06 - 0.2 % v/v, e.g. 0.075-0.125 % v/v or about 0.1 % v/v. As used herein a corresponding concentration of another tween product refers to a concentration for the product that provides the same properties as the indicated concentration of Tween20. In particularly preferred embodiments, the DNA polymerase is a Bst polymerase or a homologue thereof, such as Bst long fragment (Bst-Lf), a Bst2.0 DNA polymerase, or DNA polymerase I of *Parageobacillus yumthangensis* or a homologue thereof, such as the long fragment thereof, and the reaction buffer comprises one or more components selected from guanidine, ammonium sulphate, tween, preferably in the concentrations indicated above, preferably the reaction comprises at least two of said components, preferably all three, more preferably at least guanidine and ammonium sulphate, preferably in the concentrations indicated above.

The kit of parts of the invention may comprise a reverse transcriptase. Such kit of parts is in particular for performing a RT-LAMP reaction.

The kit of parts of the invention may comprises one or more containers, each container comprising the primer set of the invention, or a primer of the primer set, the DNA polymerase, the nucleotides and/or the reaction buffer.

The kit of parts of the invention may also include instructions for use, in particular for using the primers, and DNA polymerase, nucleotides and/or reaction buffer to amplify and detect the presence or absence of *Legionella pneumophila* in a sample.

In preferred embodiments, the kit of the invention comprises a LAMP primer set comprising a forward outer primer (F3), a backward outer primer (B3), a forward inner primer (FIP) and a backward inner primer (BIP), and optionally further comprising a loop forward primer (LoopF) and/or loop backward primer (LoopB), preferably at least one Loop primer, preferably a LoopF primer, in case of a DotB primer set of the invention and preferably a LoopB primer, in case of a MIP primer set of the invention.

In some embodiments, the kit comprises a primer set specific for the *Legionella pneumophila* DotB gene as defined herein. In preferred embodiments, the kit of the invention comprises a LAMP primer set specific for the DotB gene as depicted in figure 1, preferably a region of the DotB gene consisting of nucleotides 567-1134 as depicted in figure 1, preferably consisting of nucleotides 741-1034 as depicted in figure 1, more preferably consisting of nucleotides 769-1006 as depicted in figure 1, consisting of nucleotides 779-996 as depicted in figure 1.

In preferred embodiments:
- primer F3 comprises a consecutive stretch of at least 15 nucleotides of the sequence ATACTTCTGGTGTTGCAGAAA, more preferably of 15, 16, 17, 18, 19, 20 or 21 nucleotides of said sequence, more preferably 18, 19, 20 or 21 nucleotides of said sequence, more preferably 20 or 21 nucleotides of said sequence;
- primer B3 comprises a consecutive stretch of at least 15 nucleotides of the sequence CACTTCGTTAGGGTCTCC, more preferably of 15, 16, 17 or 18 or 21 nucleotides of said sequence, more preferably 17 or 18 nucleotides of said sequence;
- primer FIP comprises a consecutive stretch of at least 30 nucleotides or two consecutive stretches of at least 30 nucleotides in total of the sequence AATGCAAAGCCTAATTGTTTCAAGATCTTTTAGCGGGGAAGAG, more preferably of 35, 36, 37, 38, 39, 40, 41, 42 or 43 nucleotides of said sequence, more preferably 41, 42 or 43 nucleotides of said sequence;
- primer BIP comprises a consecutive stretch of at least 30 nucleotides or two consecutive stretches of at least 30 nucleotides in total of the sequence TGGTCCCAACCGTTGATGAAAGCTCCAATAGAATATCTCTCACTTC, more preferably of 35, 36, 37, 38, 39, 40, 41, 42, 43, 44, 45 or 46 nucleotides of said sequence, more preferably 43, 44, 45 or 46 nucleotides of said sequence, more preferably 45 or 46 nucleotides of said sequence, and
- primer LoopF comprises a consecutive stretch of at least 18 nucleotides of the sequence ATCGATGGTTCTTCCCAAACG, more preferably of 15, 16, 17, 18, 19, 20 or 21 nucleotides of said sequence, more preferably 18, 19, 20 or 21 nucleotides of said sequence, more preferably 20 or 21 nucleotides of said sequence.

In further preferred embodiments:
- primer F3 comprises or consists of the sequence ATACTTCTGGTGTTGCAGAAA;
- primer B3 comprises or consists of the sequence CACTTCGTTAGGGTCTCC;
- primer FIP comprises or consists of the sequence AATGCAAAGCCTAATTGTTTCAAGATCTTTTAGCGGGGAAGAG;
- primer BIP comprises or consists of the sequence TGGTCCCAACCGTTGATGAAAGCTCCAATAGAATATCTCTCACTTC, and
- primer LoopF comprises or consists of the sequence ATCGATGGTTCTTCCCAAACG.

In other embodiments, the kit comprises a primer set specific for the *Legionella pneumophila* MIP gene. In preferred embodiments, the kit of the invention comprises a LAMP primer set specific for the MIP gene, whereby
- primer F3 comprises or consists of a consecutive stretch of 15, 16, 17, 18, 19, 20, 21, 22, 23 or 24 nucleotides of the sequence CAAAGTAATCAATTCTGGAAATGG;
- primer B3 comprises or consists of a consecutive stretch of 15, 16, 17, 18, 19, 20, 21, 22 or 23 nucleotides of the sequence ACATAAATTTCCCAAGTTGATCC;
- primer FIP comprises a consecutive stretch of 30, 31, 32, 33, 34, 35, 36, 37, 38, 39, 40, 41 or 42 nucleotides of the sequence GTACCATCAATCAGACGACCAGGTTAAACCCGGAAAATCGGA;
- primer BIP comprises a consecutive stretch 30, 31, 32, 33, 34, 35, 36, 37, 38, 39 or 40 nucleotides of the sequence CCGAAAAAACTGGTAAGCCAGCTGGCATCAATTGCAAAGC, and
- primer LoopB 15, 16, 17, 18, 19, 20, 21, 22, 23 or 24 nucleotides of the sequence CAAGTTATCCCTGGATGGACAGAA.

As another example, in further preferred embodiments:
- primer F3 comprises or consists of the sequence CAAAGTAATCAATTCTGGAAATGG;
- primer B3 comprises or consists of the sequence ACATAAATTTCCCAAGTTGATCC;
- primer FIP comprises or consists of the sequence GTACCATCAATCAGACGACCAGGTTAAACCCGGAAAATCGGA;
- primer BIP comprises or consists of the sequence CCGAAAAAACTGGTAAGCCAGCTGGCATCAATTGCAAAGC, and
- primer LoopB comprises or consists of the sequence CAAGTTATCCCTGGATGGACAGAA.

In some preferred embodiments, the kit of parts comprises a primer set specific for the *Legionella pneumophila* MIP gene according to the invention and a primers set specific for the a *Legionella pneumophila* DotB gene according to the invention.

The invention further provides a use of a primer set or primer sets or kit of parts according to the invention for determining whether a sample comprises *Legionella pneumophila,* in particular using a method according to the invention.

The invention further provides a use of a primer set or primer sets or kit of parts according to the invention for detecting *Legionella pneumophila* in a sample.

The invention further provides a use of a primer set or primer sets or kit of parts according to the invention for detecting an amplification product of *Legionella pneumophila* nucleic acid in a sample.

Features may be described herein as part of the same or separate aspects or embodiments of the present invention for the purpose of clarity and a concise description. It will be appreciated by the skilled person that the scope of the invention may include embodiments having combinations of all or some of the features described herein as part of the same or separate embodiments.

The invention will be explained in more detail in the following, non-limiting examples.

### Brief description of the drawings

Figure 1: *Legionella pneumophila* DotB gene (GenBank accession number NZ_CP013742.1: nucleotides 3033061-3034194 of *Legionella pneumophila* strain Philadelphia-1 isolate AE chromosome, complete genome). Underlined sequence ATACTTCTGGTGTTGCAGAAA is the target of primer F3 in table 1, sequence ***GGAGACCCTAACGAAGTG*** (in bold and italics) is the target of primer B3 in table 1, underlined sequences TCTTGAAACAATTAGGCTTTGCATT and TCTTTTAGCGGGGAAGAG form the target of primer FIP in table 1, underlined sequences TGGTCCCAACCGTTGATGAAAG and GAAGTGAGAGATATTCTATTGGAG form the target of primer BIP in table 1, and sequence ***CGTTTGGGAAGAACCATCGAT*** (in bold and italics) is the target of primer LoopF in table 1.
Figure 2: *Legionella pneumophila* MIP gene (GenBank accession number AF095230).

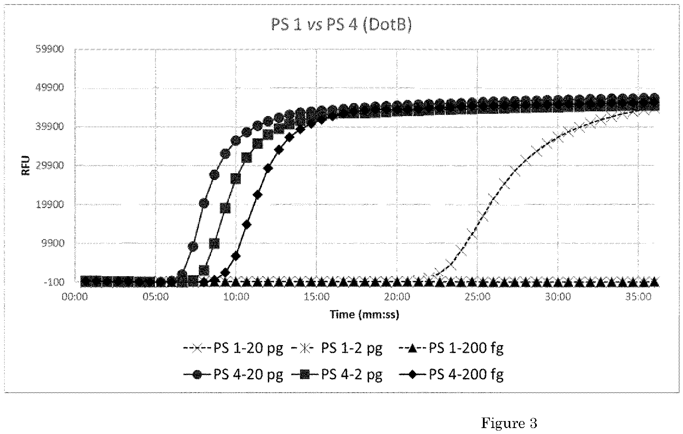

Figure 3: Comparison of a fluorometric LAMP reaction of primer set 1 (MIP primer set; Moosavian et al., 2019; Infect Drug Resist; 12:529-534. doi: 10.2147/IDR.S198099) with primer set 4 (DotB primer set of the invention). Graph displays fluorescent signal, arising from intercalation of a fluorescent dye in double strand DNA formed in the LAMP assay as a function of the assay time.

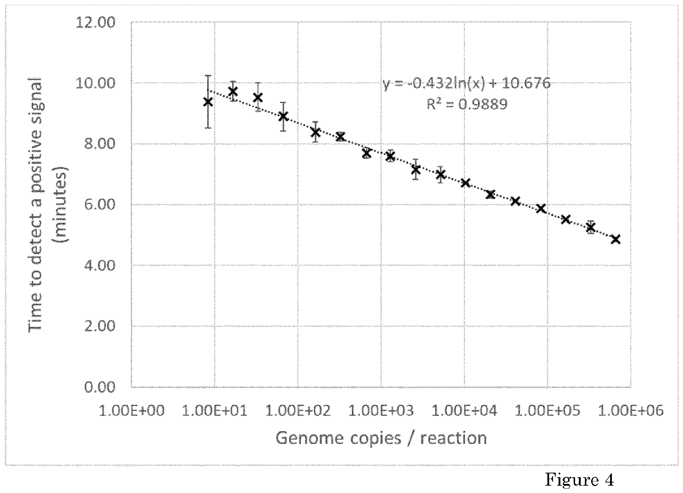

Figure 4: Template dependence of the time to detect of positive LAMP signal in the assay using the DotB primers. The log-linear fit revealed the quantitative nature of the assay established.
Figure 5: Comparison of a fluorometric LAMP reaction of MIP primer set 1 (Moosavian et al., 2019; Infect Drug Resist;12:529-534. doi: 10.2147/IDR.S198099; without loop primer) with MIP primer set 2 (primer set of the invention). Graph displays fluorescent signal, arising from intercalation of a fluorescent dye in double strand DNA formed in the LAMP assay as a function of the assay time.

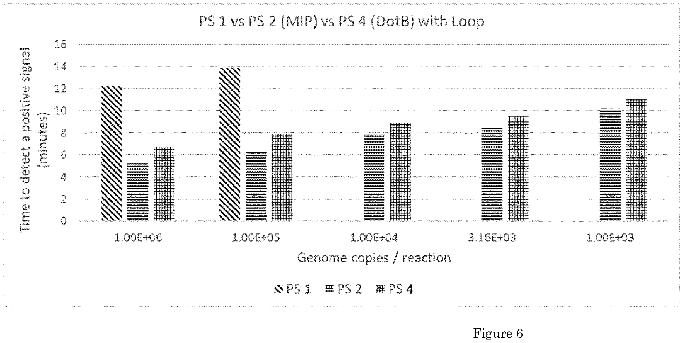

Figure 6: Comparison of a fluorometric LAMP reaction of MIP primer set 1 (Moosavian et al., 2019; Infect Drug Resist;12:529-534. doi: 10.2147/IDR.S198099; including loop primer) with MIP primer set 2 (primer set of the invention) and DotB primer set 4 (primer set of the invention). Graph displays the time to detect a positive signal for samples containing different amounts of genome copies.

### Examples

### Primer design.

Multiple primer sets were designed, targeting both the *mip* and *dotB* gene. These are Primer Sets 2 and 4, respectively, as shown in Table 1. Primers were designed using Primer Explore (https://primerexplorer.jp/e/) and GLAPD (https://doi.org/10.3389/fmicb.2019.02860). As a reference, Primer Set 1 (Moosavian et al., 2019; Infect Drug Resist;12:529-534. doi: 10.2147/IDR.S198099), targeting the Legionella pneumophila *mip* gene was used, see Table 1. Specific for this set, we additionally designed a loop primer. This loop primer is not part of the original publication.

**Table 1. LAMP primer sets.**

| Primer set | Gene | Primer | Sequence | Pos |
|---|---|---|---|---|
| Set 1 | *MIP* | F3 | ACCCGGAAAATCGGATAC | 420 |
| | | B3 | GTGGGCCATATGCAAGAC | 605 |
| | | FIP | TACCAGTTTTTCGGTACTGTACTGTCA AACACTGTCGAATATACTGGTCG | 481 |
| | | BIP | CAACGTTCCAGGTTTCACAAGTTATAAA TTTCCCAAGTTGATCCA | 512 |
| | | LoopB | GGATGGACAGAAGCTTTGCAATT | 541 |
| Set 2 | | F3 | CAAAGTAATCAATTCTGGAAATGG | 390 |
| | | B3 | ACATAAATTTCCCAAGTTGATCC | 574 |
| | | FIP | GTACCATCAATCAGACGACCAGGTTAAA CCCGGAAAATCGGA | 455 |
| | | BIP | CCGAAAAAACTGGTAAGCCAGCTGGCAT CAATTGCAAAGC | 491 |
| | | LoopB | CAAGTTATCCCTGGATGGACAGAA | 529 |
| Set 4 | *DotB Dot*/*Icm type IV secretion system* | F3 | ATACTTCTGGTGTTGCAGAAA | 778 |
| | | B3 | CACTTCGTTAGGGTCTCC | 978 |
| | | FIP | AATGCAAAGCCTAATTGTTTCAAGATCT TTTAGCGGGGAAGAG | 860 |
| | | BIP | TGGTCCCAACCGTTGATGAAAGCTCCAA TAGAATATCTCTCACTTC | 895 |
| | | LoopF | ATCGATGGTTCTTCCCAAACG | 837 |

### Standard LAMP protocol.

Lamp assay were performed in an assay mix shown in table 2 (with custom made buffer) or 3 (with commercially available buffer).

**Table 2. LAMP assay mixture.**

| Component | Final Concentration |
|---|---|
| TRIS pH 8,8 | 20 mM |
| KCL | 10 mM |
| MgSO₄ (100 mM) | 6 mM (8 mM total) |
| (NH₄)₂SO₄ | 25 mM |
| Tween20 | 0.1 % |
| Guanidine | 40 mM |
| dNTP Mix | 1.4 mM each |
| FIP/BIP Primers | 1.6 µM each |
| F3/B3 Primers | 0.2 µM each |
| Optional Loop Primers | 0.4 µM |
| DNA Polymerase (40,000 U/ml)* | 40 U |
| LAMP dye (50x) | 0.5 µl (1x) |
| DNA Sample | variable |
| Nuclease-free Water | to 25 µl |
| Total Reaction Volume | 25 µl |

**Table 3. LAMP assay mixture**

| Component | 25 µl Reaction | Final Concentration |
|---|---|---|
| 10X Xcelerator Reaction Buffer* | 2.5 µl | 1X (contains 2 mM MgSO₄) |
| MgSO₄ (100 mM) | 1.5 µl | 6 mM (8 mM total) |
| dNTP Mix (10 mM) | 3.5 µl | 1.4 mM each |
| FIP/BIP Primers (25X) | 1 µl | 1.6 µM each |
| F3/B3 Primers (25X) | 1 µl | 0.2 µM each |
| Optional Loop Primers (25X) | 1 µl | 0.4 µM |
| DNA Polymerase (40,000 U/ml)* | 1 µl | 40 U |
| LAMP dye (50x) | 0.5 µl | 0.5 µl (1x) |
| DNA Sample | variable | variable |
| Nuclease-free Water | to 25 µl | to 25 µl |
| Total Reaction Volume | 25 µl | 25 µl |

| | | |
|---|---|---|
| * The DNA polymerase and buffer were variable. | | |

Following preparation of the LAMP mixture, samples were incubated at 67°C on the Bio-Rad CFX qPCR machine during 30 minutes, with fluorescence (FAM channel) detection every 40 seconds.

### DotB LAMP

To evaluate the performance of the LAMP primer sets, experiments were executed using serial dilutions of genomic DNA extracted from *Legionella pneumophila* (ATCC 33152). In a LAMP experiment using primer set 4 (DotB), all three template concentrations tested gave a positive signal within 7 minutes. Contrastingly, primer set 1, as published (Moosavian et al., 2019; Infect Drug Resist;12:529-534. doi: 10.2147/IDR.S 198099) gave a positive signal only for the highest concentration tested (20 pg), and only gave a positive signal after 22 minutes of incubation (Figure 3).

We next evaluated the sensitivity and quantitative nature of the LAMP assay based on the DotB primer set. Results are shown in figure 4. Depending on the template concentration, a positive LAMP signal was detected between 7 and 15 minutes of incubation. Robust signals were detected up to a template level of 10-50 genome copies per reaction. A log linear fit could be made with an R^2 of 0.98, showing quantitative nature of the LAMP assay established.

### MIP LAMP

We next compared the performance of primer set 1 (Moosavian et al., 2019; Infect Drug Resist;12:529-534. doi: 10.2147/IDR.S198099) with a new primer set we designed on the basis of the *MIP* gene (primer set 2). While in assays using our MIP based primers positive signals were detected for all three concentrations tested between 6 and 10 minutes upon initiation of the reaction, for the published primer set (set 1), this was only detected for the highest concentration (20pg), and only after 22 minutes (figure 5).

New primer sets 2 (MIP) and 4 (DotB) also outperformed primer set 1 (Moosavian et al., 2019) that is supplemented with a custom made loop primer. As shown in figure 6, the time required to detect a signal is reduced by about 50% with the new MIP and DotB primer sets 2 and 4 as compared to the known MIP primer set 1. For lower concentrations genetic material, detection was not possible with primer set 1 with additional loop primer, whereas primer sets 2 and 4 provided a signal after 8-11 minutes.

### Comparative analysis of DotA primersets

We next performed a comparative analysis of published DotA primers (CN106755302A) and the newly developed DotB and MIP primer sets in which 11 different *Legionella pneumophila* isolates were used.

As shown in table 4, with the DotA primer sets only 5 of the 11 isolates could be detected. With the newly designed dotB primer set 10 out of 11 isolates could be detected, and with the newly designed MIP 10 out of 11 isolates could be detected. The combination of the new DotB and MIP primer sets, was able to detect all Legionella isolates.

Hence, the new DotB and MIP primer sets outperform previously known DotA and MIP LAMP primers sets. Further, the combination of the DotB and MIP primer sets allow both a very fast detection and a broad taxonomic representation. Using the previously known DotA primer set leads to many false negative results because a large part of the *Legionella* isolates cannot be detected.

**Table 4. Detection of 11 Legionella pneumophila isolates using different LAMP primer sets.**

| Strain number | (sero)type | type | Dot A PSA1 | PSA2 | PSA3 | TNO MIP PS2 | DotB PS4 |
|---|---|---|---|---|---|---|---|
| TTC 99.0454 | sero 10 | n.b. | - | - | - | + | + |
| TTC 99.0459 | sero 8 | n.b. | - | - | - | + | + |
| TTC 99.0516 | sero 1 | Ia | - | - | - | + | + |
| TTC 99.0524 | sero 6 | Ib | + | + | + | + | + |
| TTC 99.0527 | sero 6 | Ib | + | + | + | + | + |
| TTC 99.0528 | sero 1 | III | - | - | + | + | - |
| TTC 99.0533 | sero 1 | IV | + | + | + | + | + |
| TTC 99.0538 | sero 1 | V | - | - | - | + | + |
| 2007.49-1 | Philadelphia | | + | + | + | + | + |
| 2007.50-1 | Paris | | - | - | - | + | + |
| 2007.51-1 | Lens | | + | + | + | - | + |

## Claims

1. A method for determining whether a sample comprises *Legionella pneumophila,* the method comprising performing an isothermal nucleic acid amplification (iNAAT) reaction with said sample with a primer set specific for the *Legionella pneumophila* DotB gene and determining whether the sample comprises an amplification product of the amplification reaction.

2. A method of detecting an amplification product in a sample, the method comprising performing an isothermal nucleic acid amplification (iNAAT) reaction with said sample with a primer set specific for the *Legionella pneumophila* DotB gene and determining whether the sample comprises an amplification product of the amplification reaction.

3. The method according to claim 1 or 2, wherein said isothermal nucleic acid amplification reaction is a loop-mediated isothermal amplification (LAMP) reaction.

4. The method according to any one of the preceding claims wherein said amplification reaction is performed with a primer set specific for a region of the DotB gene consisting of nucleotides 567-1134 as depicted in figure 1, preferably consisting of nucleotides 741-1034 as depicted in figure 1, more preferably consisting of nucleotides 769-1006 as depicted in figure 1, consisting of nucleotides 779-996 as depicted in figure 1.

5. The method according to any one of the preceding claims wherein said amplification reaction is performed with a LAMP primer set comprising a forward outer primer (F3), a backward outer primer (B3), a forward inner primer (FIP) and a backward inner primer (BIP), and optionally further comprising a loop forward primer (LoopF) and/or loop backward primer (LoopB), preferably at least one Loop primer, more preferably a LoopF primer, and wherein:
- primer F3 comprises a consecutive stretch of at least 15 nucleotides of the sequence ATACTTCTGGTGTTGCAGAAA;
- primer B3 comprises a consecutive stretch of at least 15 nucleotides of the sequence CACTTCGTTAGGGTCTCC;
- primer FIP comprises a consecutive stretch of at least 30 nucleotides or two consecutive stretches of at least 30 nucleotides in total of the sequence AATGCAAAGCCTAATTGTTTCAAGATCTTTTAGCGGGGAAGAG;
- primer BIP comprises a consecutive stretch of at least 30 nucleotides or two consecutive stretches of at least 30 nucleotides in total of the sequence TGGTCCCAACCGTTGATGAAAGCTCCAATAGAATATCTCTCACTTC, and optionally
- primer LoopF comprises a consecutive stretch of at least 15 nucleotides of the sequence ATCGATGGTTCTTCCCAAACG.

6. The method according to any one of the preceding claims wherein:
- primer F3 comprises the sequence ATACTTCTGGTGTTGCAGAAA;
- primer B3 comprises the sequence CACTTCGTTAGGGTCTCC;
- primer FIP comprises the sequence AATGCAAAGCCTAATTGTTTCAAGATCTTTTAGCGGGGAAGAG;
- primer BIP comprises the sequence TGGTCCCAACCGTTGATGAAAGCTCCAATAGAATATCTCTCACTTC, and optionally
- primer LoopF comprises the sequence ATCGATGGTTCTTCCCAAACG.

7. A method for determining whether a sample comprises *Legionella pneumophila,* the method comprising performing an isothermal nucleic acid amplification (iNAAT) reaction with said sample with a primer set specific for the *Legionella pneumophila* MIP gene, wherein said primer set comprises a forward outer primer (F3), a backward outer primer (B3), a forward inner primer (FIP) and a backward inner primer (BIP), and optionally further comprising a loop forward primer (LoopF) and/or loop backward primer (LoopB), preferably at least one Loop primer, more preferably a LoopB primer, and determining whether the sample comprises an amplification product of the amplification reaction, wherein:
- primer F3 comprises a consecutive stretch of at least 15 nucleotides of the sequence CAAAGTAATCAATTCTGGAAATGG;
- primer B3 comprises a consecutive stretch of at least 15 nucleotides of the sequence ACATAAATTTCCCAAGTTGATCC;
- primer FIP comprises a consecutive stretch of at least 30 nucleotides or two consecutive stretches of at least 30 nucleotides in total of the sequence GTACCATCAATCAGACGACCAGGTTAAACCCGGAAAATCGGA;
- primer BIP comprises a consecutive stretch of at least 30 nucleotides or two consecutive stretches of at least 30 nucleotides in total of the sequence CCGAAAAAACTGGTAAGCCAGCTGGCATCAATTGCAAAGC, and optionally
- primer LoopB comprises a consecutive stretch of at least 15 nucleotides of the sequence CAAGTTATCCCTGGATGGACAGAA.

8. A method of detecting an amplification product in a sample, the method comprising performing an isothermal nucleic acid amplification (iNAAT) reaction with said sample with a primer set specific for the *Legionella pneumophila* MIP gene, wherein said primer set comprises a forward outer primer (F3), a backward outer primer (B3), a forward inner primer (FIP) and a backward inner primer (BIP), and optionally further comprising a loop forward primer (LoopF) and/or loop backward primer (LoopB), preferably at least one Loop primer, more preferably a LoopB primer, and determining whether the sample comprises an amplification product of the amplification reaction, wherein
- primer F3 comprises a consecutive stretch of at least 15 nucleotides of the sequence CAAAGTAATCAATTCTGGAAATGG;
- primer B3 comprises a consecutive stretch of at least 15 nucleotides of the sequence ACATAAATTTCCCAAGTTGATCC;
- primer FIP comprises a consecutive stretch of at least 30 nucleotides or two consecutive stretches of at least 30 nucleotides in total of the sequence GTACCATCAATCAGACGACCAGGTTAAACCCGGAAAATCGGA;
- primer BIP comprises a consecutive stretch of at least 30 nucleotides or two consecutive stretches of at least 30 nucleotides in total of the sequence CCGAAAAAACTGGTAAGCCAGCTGGCATCAATTGCAAAGC, and optionally
- primer LoopB comprises a consecutive stretch of at least 15 nucleotides of the sequence CAAGTTATCCCTGGATGGACAGAA.

9. The method according to any one of the preceding claims wherein the sample is an environmental sample or a biological sample, in particular a sample obtained from an individual.

10. A loop-mediated isothermal amplification (LAMP) primer set specific for *Legionella pneumophila* DotB gene, the primer set comprising a forward outer primer (F3), a backward outer primer (B3), a forward inner primer (FIP) and a backward inner primer (BIP), and optionally a loop forward primer (LoopF) and/or loop backward primer (LoopB), preferably at least one Loop primer, more preferably a LoopF primer,.

11. The LAMP primer set according to claim 10 wherein said primer set is specific for a region of the DotB gene consisting of nucleotides 567-1134 as depicted in figure 1, preferably consisting of nucleotides 741-1034 as depicted in figure 1, more preferably consisting of nucleotides 769-1006 as depicted in figure 1, consisting of nucleotides 779-996 as depicted in figure 1.

12. The LAMP primer set according to claim 10 or 11 wherein:
- primer F3 comprises a consecutive stretch of at least 15 nucleotides of the sequence ATACTTCTGGTGTTGCAGAAA;
- primer B3 comprises a consecutive stretch of at least 15 nucleotides of the sequence CACTTCGTTAGGGTCTCC;
- primer FIP comprises a consecutive stretch of at least 30 nucleotides or two consecutive stretches of at least 30 nucleotides in total of the sequence AATGCAAAGCCTAATTGTTTCAAGATCTTTTAGCGGGGAAGAG;
- primer BIP comprises a consecutive stretch of at least 30 nucleotides or two consecutive stretches of at least 30 nucleotides in total of the sequence TGGTCCCAACCGTTGATGAAAGCTCCAATAGAATATCTCTCACTTC, and optionally
- primer LoopF comprises a consecutive stretch of at least 18 nucleotides of the sequence ATCGATGGTTCTTCCCAAACG.

13. A loop-mediated isothermal amplification (LAMP) primer set specific for *Legionella pneumophila* MIP gene, the primer set comprising a forward outer primer (F3), a backward outer primer (B3), a forward inner primer (FIP) and a backward inner primer (BIP), and optionally a loop forward primer (LoopF) and/or loop backward primer (LoopB), wherein:
- primer F3 comprises a consecutive stretch of at least 15 nucleotides of the sequence CAAAGTAATCAATTCTGGAAATGG;
- primer B3 comprises a consecutive stretch of at least 15 nucleotides of the sequence ACATAAATTTCCCAAGTTGATCC;
- primer FIP comprises a consecutive stretch of at least 30 nucleotides or two consecutive stretches of at least 30 nucleotides in total of the sequence GTACCATCAATCAGACGACCAGGTTAAACCCGGAAAATCGGA;
- primer BIP comprises a consecutive stretch of at least 30 nucleotides or two consecutive stretches of at least 30 nucleotides in total of the sequence CCGAAAAAACTGGTAAGCCAGCTGGCATCAATTGCAAAGC, and optionally
- primer LoopB comprises a consecutive stretch of at least 15 nucleotides of the sequence CAAGTTATCCCTGGATGGACAGAA.

14. A kit of parts comprising the LAMP primer set according to any one of claims 10-13, further comprising a DNA polymerase, nucleotides and/or a reaction buffer.
